# EUROPEAN PATENT SPECIFICATION

(11) **EP 2 558 574 B1**
(45) Date of publication and mention of the grant of the patent: **18.03.2015**
(21) Application number: 11769488.5
(22) Date of filing: 13.04.2011
(51) Int. Cl.: C12N 15/87, C12N 15/85, C12N 15/90, G01N 33/50, C07K 14/47

(54) **USE OF ENDOGENOUS PROMOTERS TO EXPRESS HETEROLOGOUS PROTEINS**
VERWENDUNG ENDOGENER PROMOTOREN ZUR EXPRESSION HETEROLOGER PROTEINE
UTILISATION DE PROMOTEURS ENDOGÈNES POUR EXPRIMER DES PROTÉINES HÉTÉROLOGUES

(30) Priority: 12.01.2011 US 431957 P; 01.11.2010 US 408856 P; 07.10.2010 US 390668 P; 23.07.2010 US 367017 P; 13.04.2010 US 323698 P; 13.04.2010 US 323719 P; 13.04.2010 US 323702 P
(43) Date of publication of application: 20.02.2013
(73) Proprietor: Sigma-Aldrich Co. LLC, St. Louis, MO 63103 (US)
(72) Inventor: DAVIS, Greg, St. Louis, Missouri 63103 (US); MALKOV, Dmitry, St. Louis, Missouri 63103 (US); ZENSER, Nathan, St. Louis, Missouri 63103 (US)
(74) Representative: Turner, Rhiannon Rosalind
(86) International application number: PCT/US2011/032216
(87) International publication number: WO 2011/130345

(56) References cited:
- US-A1- 2006 063 231
- US-A1- 2009 117 617
- US-A1- 2009 117 617
- US-A1- 2010 047 805
- VIPULA K SHUKLA ET AL: "Precise genome modification in the crop species Zea mays using zinc-finger nucleases", NATURE: INTERNATIONAL WEEKLY JOURNAL OF SCIENCE (AND SUPPLEMENTARY INFORMATION), NATURE PUBLISHING GROUP, UNITED KINGDOM, vol. 459, no. 7245, 21 May 2009 (2009-05-21), page 437, XP002626698, ISSN: 0028-0836, DOI: 10.1038/NATURE07992 [retrieved on 2009-04-29]
- PA RADCLIFFE AND KA MITROPHANOUS: "Multiple gene products from a single vector: 'self-cleaving' 2A peptides", GENE THERAPY, NATURE PUBLISHING GROUP, GB, vol. 11, no. 23, 1 December 2004 (2004-12-01), pages 1673-1674, XP008138931, ISSN: 0969-7128, DOI: 10.1038/SJ.GT.3302361
- DATABASE GENBANK [Online] 05 April 2010 'Homo sapiens tubulin, alpha 1b (TUBA1 B), mRNA.', XP008161866
- DATABASE GENBANK [Online] 28 February 2010 'Homo sapiens actin, beta (ACTB), mRNA.', XP008161867
- DE FELIPE ET AL.: 'E unum pluribus: multiple proteins from a self-processing polyprotein.' TRENDS BIOTECHNOL vol. 24, no. 2, 2006, pages 68 - 75, XP027921654
- URNOV ET AL.: 'Genome editing with engineered zinc finger nucleases.' NATURE REV GENET vol. 11, no. 9, September 2010, pages 636 - 646, XP008150557

### SEQUENCE LISTING

<110> SIGMA-ALDRICH CO.
   DAVIS, Greg
   MALKOV, Dmitry
   ZENSER, Nathan
<120> USE OF ENDOGENOUS PROMOTERS TO EXPRESS HETEROLOGOUS PROTEINS
<130> 047497-424742
<150> US 61/323,702
   <151> 2010-04-13
<150> US 61/367,017
   <151> 2010-07-23
<150> US 61/390,668
   <151> 2010-10-07
<150> US 61/408,856
   <151> 2010-11-01
<150> US 61/431,957
   <151> 2011-01-12
<150> US 61/323,719
   <151> 2010-04-13
<150> US 61/323,698
   <151> 2010-04-13
<160> 12
<170> PatentIn version 3.5
<210> 1
   <211> 15
   <212> DNA
   <213> Homo sapiens
<400> 1
   cttcgcctcc taatc 15
<210> 2
   <211> 15
   <212> DNA
   <213> Homo sapiens
<400> 2
   cactatggtg agtaa 15
<210> 3
   <211> 18
   <212> DNA
   <213> Homo sapiens
<400> 3
   gtcgtcgaca acggctcc 18
<210> 4
   <211> 19
   <212> DNA
   <213> Homo sapiens
<400> 4
   tgcaaggccg gcttcgcgg 19
<210> 5
   <211> 15
   <212> DNA
   <213> Homo sapiens
<400> 5
   cctcgccgcc ccgct 15
<210> 6
   <211> 16
   <212> DNA
   <213> Homo sapiens
<400> 6
   gccgcccgcc atggcg 16
<210> 7
   <211> 16
   <212> DNA
   <213> Cricetulus griseus
<400> 7
   cttttgtgcc ctgata 16
<210> 8
   <211> 18
   <212> DNA
   <213> cricetulus griseus
<400> 8
   gccatggatg acgatatc 18
<210> 9
   <211> 46
   <212> DNA
   <213> Homo sapiens
<400> 9
   tgtcgccttc gcctcctaat ccctagccac tatggtgagt aagccg 46
<210> 10
   <211> 69
   <212> DNA
   <213> Homo sapiens
<400> 10
   atggatgatg atatcgccgc gctcgtcgtc gacaacggct ccggcatgtg caaggccggc 60
   ttcgcgggc 69
<210> 11
   <211> 37
   <212> DNA
   <213> Homo sapiens
<400> 11
   cctcgccgcc ccgctgtctc cgccgcccgc catggcg 37
<210> 12
   <211> 119
   <212> DNA
   <213> Cricetulus griseus
<400> 12
   cgaccctctt ttgtgccctg atatagttcg ccatggatga cgatatcgct gcgctcgttg 60
   tcgacaacgg ctccggcatg tgcaaagccg gcttcgcggg cgacgatgct ccccgggcc 119

## Description

### CROSS-REFERENCE TO RELATED APPLICATIONS

This application claims the priority of US provisional application number 61/323,702, filed April 13, 2010, US provisional application number 61/323,719, filed April 13, 2010, US provisional application number 61/323,698, filed April 13, 2010, US provisional application number 61/367,017, filed July 23, 2010, US provisional application number 61/390,668, filed October 7, 2010, US provisional application number 61/408,856, filed November 1, 2010, and US provisional application number 61/431,957, filed January 12, 2011, each of which is hereby incorporated by reference in its entirety.

### FIELD OF THE INVENTION

The invention generally relates to the use of endogenous transcriptional control pathways to regulate the expression of heterologous proteins.

### BACKGROUND OF THE INVENTION

Expressing recombinant proteins in mammalian cells presents several challenges. First, the heterologous DNA needs to be stably incorporated into the mammalian genome. Many methods, such as viral and non-viral transfection procedures, integrate DNA randomly in the genome creating off-target effects and variable expression. While recombination-based strategies (e.g., Cre-*loxP* or Flp-*FRT*) enable the insertion of heterologous DNA into defined locations, cell lines comprising the specific recombination sites must first be created and characterized. This is not only a time-consuming process, but also the recombinase sites are placed randomly. Second, the heterologous DNA needs to be linked to a strong promoter. Generally, promoters of viral origin are used but these are susceptible to silencing. It would be desirable to be able to precisely target and integrate heterologous DNA into the mammalian genome such that it is expressed from a strong endogenous promoter.

### SUMMARY OF THE INVENTION

Provided herein are methods for integrating sequences encoding heterologous proteins into targeted locations in the genome such that endogenous regulatory systems regulated the expression of the heterologous proteins.

One aspect of the present disclosure encompasses a method for integrating a sequence encoding at least one heterologous protein in a chromosome of a cell such that expression of the at least one heterologous protein is regulated by an endogenous regulatory system. The method comprises introducing into the cell (i) at least one targeting endonuclease or nucleic acid encoding a targeting endonuclease, wherein the targeting endonuclease is able to bind a target sequence and cut a cleavage site in a chromosomal sequence that codes an endogenous protein; and (ii) at least one donor polynucleotide comprising the sequence encoding the at least one heterologous protein that is linked to a sequence encoding a 2A peptide to form a heterologous protein coding sequence. The heterologous protein coding sequence in the donor polynucleotide is flanked by an upstream sequence and a downstream sequence, which have substantial sequence identity with either side of the cleavage site in the chromosomal sequence. The method further comprises maintaining the cell under conditions such that a double-stranded break introduced into the chromosomal sequence by the targeting endonuclease is repaired by a homology-directed repair process such that the heterologous protein coding sequence in the donor polynucleotide is integrated in-frame into the targeted chromosomal sequence, whereby expression of the at least one heterologous proteins is regulated by the endogenous regulatory system that regulated expression of the endogenous protein.

Another aspect provides a cell comprising a chromosomally integrated sequence encoding at least one heterologous protein, wherein the sequence encoding the at least one heterologous protein is integrated in-frame with a chromosomal sequence encoding an endogenous protein. Expression of the at least one heterologous protein is coordinately controlled with expression of the endogenous protein in the cell.

Still another aspect of the disclosure encompasses donor polynucleotide. The donor polynucleotide comprises a sequence encoding at least one heterologous protein that is linked to a sequence encoding a 2A peptide to form a heterologous protein coding sequence. Additionally, the heterologous protein coding sequence in the donor polynucleotide is flanked by an upstream sequence and a downstream sequence that share substantial sequence identity with either side of a cleavage site in a chromosomal sequence of a target cell.

A further aspect provides a kit for integrating a sequence encoding at least one heterologous protein into a chromosomal sequence encoding an endogenous protein. The kit comprises at least one nucleic acid encoding a zinc finger nuclease, wherein the zinc finger nuclease is able to bind a target sequence and cut a cleavage site in the chromosomal sequence. The kit further comprises at least one donor polynucleotide. The donor polynucleotide comprises the sequence encoding the at least one heterologous protein that is linked to a sequence encoding a 2A peptide to form a heterologous protein coding sequence, wherein the heterologous protein sequence is flanked by an upstream sequence and a downstream sequence that have substantial sequence identity with either side of a cleavage site in the chromosomal sequence of a cell.

Yet another aspect provides a method for using an endogenous regulatory system to regulate expression of at least one heterologous protein. The method comprises providing a cell comprising a chromosomally integrated sequence encoding at least one heterologous protein linked to a sequence encoding a 2A peptide, wherein the sequences encoding the heterologous protein and the 2A peptide are integrated in-frame with a chromosomal sequence encoding an endogenous protein. The method further comprises maintaining the cell under conditions such that activation of the endogenous regulatory system produces one transcript encoding the heterologous protein, the 2A peptide, and the endogenous protein, wherein the 2A peptide disrupts translation such that each of the heterologous and endogenous proteins is produced as a discrete entity.

Other aspects and features of the disclosure are described more thoroughly below.

### REFERENCE TO COLOR FIGURES

The application file contains at least one photograph executed in color. Copies of this patent application publication with color photographs will be provided by the Office upon request and payment of the necessary fee.

### BRIEF DESCRIPTION OF THE FIGURES

**FIG. 1** depicts targeted integration at the human *TUBA1B* locus. (A) is a schematic showing the chromosome sequence (SEQ ID NO:9) at the target region for integration of the heterologous coding sequence, ZFN binding sites (boxed regions) on the chromosome target region, the ZFN cut site (yellow arrow), and the integration site (green arrow). The site of integration was 7 bp downstream of the cut site. (B) presents schematics of the *TUBA1B* locus, site of integration, design of the SH2 biosensor, and the proteins expressed after successful integration.
**FIG. 2** depicts the map of a donor plasmid comprising the SH2 biosensor sequence flanked by *TUBA1A* sequences at the target region.
**FIG. 3** presents an image of a Western blot of wild-type and cells with a targeted integration.
**FIG. 4** presents differential interference contrast (DIC) and fluorescence microscopy images of individual isolated cell clones expressing the GFP-2xSH2(Grb2)-2A protein. Fluorescent images show a time course of biosensor translocation after exposure to 100 ng/mL of EGF.
**FIG. 5** depicts targeted integration at the human *ACTB* locus. Shown is the chromosome sequence (SEQ ID NO:10) at the target region for integration of the heterologous coding sequence, ZFN binding sites (yellow sequence) in the chromosome target region, the ZFN cut site (upper, yellow arrow), and the tag sequence integration site (lower, green/yellow arrow).
**FIG. 6** presents the map of a donor plasmid comprising the SH2 biosensor sequence flanked by *ACTB* sequences at the target region.
**FIG. 7** depicts fluorescence microscopy images of individual isolated cell clones expressing GFP-2xSH2(Grb2)-2A (upper panels) and RFP-β-actin (lower panels). Presented is a time course after exposure to 100 ng/mL of EGF.
**FIG. 8** depicts targeted integration at the *LMNB*1 locus. Shown in chromosome sequence (SEQ ID NO:11) at the target region for integration of the heterologous coding sequence, ZFN binding sites (yellow sequence) in the chromosome target region, the ZFN cut site (yellow arrow), and the tag sequence integration site (green arrow).
**FIG. 9** shows the site of targeted integration in the *ACTB* locus of Chinese hamster ovary (CHO) cells. Shown is the chromosome sequence (SEQ ID NO:12) at the target region for integration of the heterologous coding sequence, ZFN binding sites (boxed regions), the ZFN cleavage site, and the targeted integration site.

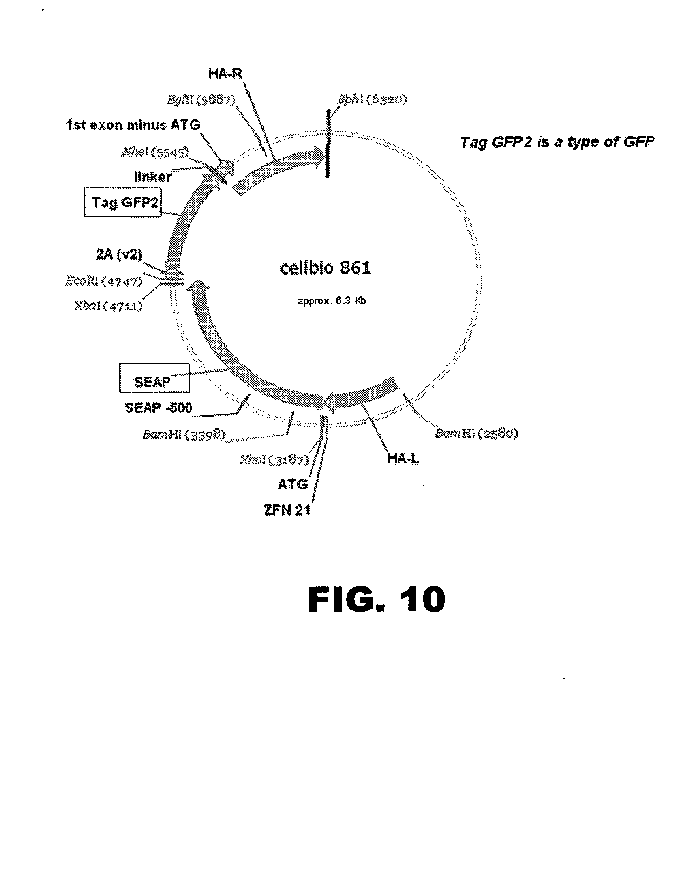

**FIG. 10** depicts the map of a donor plasmid comprising the SEAP-2A-GFP sequence flanked by CHO *ACTB* sequences upstream and downstream of the ZFN cleavage site.
**FIG. 11** depicts junction PCR analysis of the targeted integration of the SEAP-2A-GFP sequence into the *ACTB* locus of CHO cells. The amplified fragment is the expected size.

### DETAILED DESCRIPTION OF THE INVENTION

Among the various aspects disclosed herein is a method for integrating a sequence encoding at least one heterologous protein into a targeted location in a cellular chromosome such that expression of the heterologous protein(s) is regulated by an endogenous transcriptional control system. Thus, rather than using an exogenous (e.g., viral) promoter, expression is regulated by an endogenous system comprising not only a promoter sequence but other cis regulatory elements located upstream and downstream of the transcriptional start site. Advantageously, an endogenous system is not susceptible to silencing effects. Moreover, by linking the heterologous coding sequence to a 2A peptide coding sequence, individual heterologous and endogenous proteins are made during translation. The sequence encoding the heterologous protein(s) is integrated into a targeted chromosomal location by a targeting endonuclease genome editing process. Also provided herein are cells comprising a chromosomally integrated sequence encoding at least one heterologous protein that is operably linked to an endogenous regulatory system and methods for using an endogenous regulatory system to express the heterologous protein(s).

### (I) Cell Comprising Heterologous Sequence Whose Expression is Regulated by Endogenous Regulatory System

One aspect of the present disclosure encompasses a cell comprising a chromosomally integrated sequence encoding at least one heterologous protein whose expression is regulated by an endogenous regulatory system. In particular, the sequence encoding the heterologous protein(s) is integrated in-frame with an endogenous chromosomal sequence encoding an endogenous protein. A targeting endonuclease genome editing mediated process is used to target and integrate the heterologous coding sequence to the endogenous chromosomal sequence of interest. Additionally, the heterologous coding sequence is linked to a 2A peptide coding sequence. Upon activation of transcription, the heterologous and endogenous sequences are transcribed as a single transcript. During translation, the 2A peptide disrupts translation such that the heterologous protein is "cleaved" from the endogenous protein, thereby permitting the coordinated synthesis of more than one protein from one open reading frame.

### (a) heterologous sequence

The identity of the heterologous protein or proteins can and will vary. In general, a sequence encoding any protein may be integrated into a targeted chromosomal location. The heterologous protein may be a naturally occurring protein or fragment thereof, a recombinant protein, a fusion protein, a reporter protein, a tagged protein, a wild-type protein, a therapeutic protein, a diagnostic protein, an antibody, and so forth. For example, the heterologous protein(s) may be heavy chains or light chains of an antibody. The heterologous protein(s) may be derived from a variety of sources including, e.g., mammals, vertebrates, invertebrates, plants, microbes, bacteria, and archaebacteria.

In some embodiments, a sequence encoding more than one heterologous protein may be integrated into the chromosomal sequence. For example, a sequence encoding two, three, four, or more heterologous proteins may be integrated into the chromosomal sequence such that an endogenous regulatory system regulates the expression of two, three, four, or more heterologous proteins.

In general, the sequence encoding the heterologous protein(s) will be codon optimized for optimal expression in the cell of interest. The sequence encoding the heterologous protein(s) may comprise exonic (or protein coding) sequence. Alternatively, the sequence encoding the heterologous protein may comprise intronic sequence as well exonic sequence.

As mentioned above, the sequence encoding the heterologous protein is linked to a 2A peptide. As used herein, the term "2A peptide" refers to any 2A peptide or fragment thereof, any 2A-like peptide or fragment thereof, or an artificial peptide comprising the requisite amino acids. The 2A peptide was originally characterized in positive-strand RNA viruses, which produce a polyprotein that is "cleaved" during translation into mature individual proteins. More specifically, the 2A peptide region (∼20 amino acids) mediates "cleavage" at its own C-terminus to release itself from the 2B region of the polyprotein. 2A peptide sequences terminate with a glycine and a proline residue. During translation of a 2A peptide, the ribosome pauses after the glycine residue, resulting in release of the nascent polypeptide chain. Translation resumes, with the proline residue of the 2A sequence becoming the first amino acid of the downstream protein.

The 2A peptide coding sequence that is linked to the heterologous coding sequence may code for a full length 2A peptide. Alternatively, it may code for a C-terminal fragment of a 2A peptide. The C-terminal fragment may comprise about 19, 18,17, 16, 15, 14, 13, 12, 11, 10, 9, 8, 7, 6, 5, or 4 amino acid residues of the C-terminal end.

The sequence encoding the 2A peptide may be linked to the 5' end or 3' end of the sequence coding the heterologous protein. In embodiments in which the heterologous sequence is integrated near the beginning of an endogenous coding sequence, the 2A peptide sequence will be linked to the 3' end of the sequence encoding the heterologous protein(s). Accordingly, the resultant mRNA has the following orientation: 5'-(heterologous protein-2A peptide)ₙ-endogenous protein-3', wherein n represents the number of heterologous proteins. In embodiments in which the heterologous sequence is integrated near the end of an endogenous coding sequence, the 2A peptide sequence will be linked to the 5' end of the sequence encoding the heterologous protein(s). Thus, the resultant mRNA has the following orientation: 5'-endogenous protein-(2A peptide-heterologous protein)ₙ-3', wherein n is as defined above.

### (b) endogenous regulatory system

In general, the endogenous chromosomal sequence that is chosen for integration of the heterologous sequence will depend upon the desired expression properties. As used herein, the term "endogenous regulatory system" refers to the chromosomal sequences (i.e., transcriptional control elements such as promoter, enhancers, and the like) and the regulatory control proteins (i.e., general and specific transcription factors) that work together to regulate transcription of a chromosomal sequence. The target sequence comprises the transcriptional control sequence elements (e.g., promoter and other control elements) as well as the chromosomal sequence that is transcribed (i.e., untranslated and translated sequences). Although expression of protein coding sequences may be regulated by a variety of sequence elements, the term "promoter" is used below for ease of discussion.

In some embodiments it may be desirable to target an endogenous target sequence that utilizes a constitutive promoter. Constitutive promoters tend to be active in many types of cells. Non-limiting examples of suitable constitutive promoters include those regulating the expression of cytoskeletal proteins such as α-tubulin, β-tubulin, alpha-actin, beta-actin, and so forth; ubiquitous cellular proteins such as histone proteins, ribosomal proteins, translation factors, transcription factors, cell cycle proteins, proteasomal proteins, and the like; enzymes involved in amino acid, carbohydrate, or lipid metabolism, the citric acid cycle, mitochondrial function, and so forth. Some constitutive promoters may also be termed strong promoters in that their activation leads to high levels of gene product.

In other embodiments, expression may be desired in a particular cell type, such as, e.g., muscle cells, neural cells, hepatic cells, pancreatic beta cells, cardiac cells, mammary gland cells, and so forth. Those of skill in the art are familiar with appropriate cell-specific promoter that may be used for cell-specific or tissue-specific expression. In still another embodiment, regulatable or inducible expression may be desired. Suitable inducible promoters include those regulated by steroid hormones, growth factors, metal ions, heat shock, and so forth.

Non-limiting examples of exemplary human or mammalian expression regulatory systems include those encoding and regulating the expression of tubulin, actin, or lamin proteins.

In general, the sequence encoding the heterologous protein is integrated in-frame with the endogenous sequence coding the protein of interest. The heterologous sequence may be integrated in-frame after the start codon of the endogenous coding sequence. Alternatively, the heterologous sequence may be integrated in-frame before the stop codon of the endogenous coding sequence.

### (c) cells

The type of cell comprising the chromosomally integrated sequence encoding heterologous protein(s) described above can and will vary. In general, the cell will be a eukaryotic cell. In some instances, the cell may be a primary cell, a cultured cell, or immortal cell line cell. Suitable cells include fungi or yeast, such as *Pichia, Saccharomyces,* or *Schizosaccharomyces*; insect cells, such as SF9 cells from *Spodoptera frugiperda* or S2 cells from *Drosophila melanogaster*; and animal cells, such as mouse, rat, hamster, non-human primate, or human cells. Exemplary cells are mammalian. The mammalian cells may be primary cells. In general, any primary cell that is sensitive to double strand breaks may be used. The cells may be of a variety of cell types, e.g., fibroblast, myoblast, T or B cell, macrophage, epithelial cell, and so forth.

When mammalian cell lines are used, the cell line may be any established cell line or a primary cell line that is not yet described. The cell line may be adherent or non-adherent, or the cell line may be grown under conditions that encourage adherent, non-adherent or organotypic growth using standard techniques known to individuals skilled in the art. Non-limiting examples of suitable mammalian cell lines include Chinese hamster ovary (CHO) cells, monkey kidney CVI line transformed by SV40 (COS7), human embryonic kidney line 293, baby hamster kidney cells (BHK), mouse sertoli cells (TM4), monkey kidney cells (CVI-76), African green monkey kidney cells (VERO), human cervical carcinoma cells (HeLa), canine kidney cells (MDCK), buffalo rat liver cells (BRL 3A), human lung cells (W138), human liver cells (Hep G2), mouse mammary tumor cells (MMT), rat hepatoma cells (HTC), HIH/3T3 cells, the human U2-OS osteosarcoma cell line, the human A549 cell line, the human K562 cell line, the human HEK293 cell lines, the human HEK293T cell line, and TRI cells. For an extensive list of mammalian cell lines, those of ordinary skill in the art may refer to the American Type Culture Collection catalog (ATCC^{®}, Mamassas, VA).

In still other embodiments, the cell may be a stem cell. Suitable stem cells include without limit embryonic stem cells, ES-like stem cells, fetal stem cells, adult stem cells, pluripotent stem cells, induced pluripotent stem cells, multipotent stem cells, oligopotent stem cells, and unipotent stem cells.

In further embodiments, the cell may be a one-cell embryo. The embryo may be a vertebrate or an invertebrate. Suitable vertebrates include mammals, birds, reptiles, amphibians, and fish. Examples of suitable mammals include without limit rodents, companion animals, livestock, and non-primates. Non-limiting examples of rodents include mice, rats, hamsters, gerbils, and guinea pigs. Suitable companion animals include but are not limited to cats, dogs, rabbits, hedgehogs, and ferrets. Non-limiting examples of livestock include horses, goats, sheep, swine, cattle, llamas, and alpacas. Suitable non-primates include but are not limited to capuchin monkeys, chimpanzees, lemurs, macaques, marmosets, tamarins, spider monkeys, squirrel monkeys, and vervet monkeys. Non-limiting examples of birds include chickens, turkeys, ducks, and geese. Alternatively, the animal may be an invertebrate such as an insect, a nematode, and the like. Non-limiting examples of insects include *Drosophila* and mosquitoes.

### (II) Method for Integrating Heterologous Coding Sequence

Another aspect of the disclosure provides a method for integrating a nucleic acid encoding the at least one heterologous protein into a targeted location in a cellular chromosome such that expression of the heterologous protein(s) is controlled by an endogenous regulatory system. The method comprises using a targeting endonuclease to mediate integration of the heterologous coding sequence in-frame with an endogenous coding sequence. More specifically, the method comprises introducing into the cell at least one targeting endonuclease or nucleic acid encoding a targeting endonuclease and at least one donor polynucleotide comprising the heterologous coding sequence. The method further comprises maintaining the cell under conditions such that a double-stranded break introduced into the endogenous chromosomal sequence by the targeting endonuclease is repaired by a homology-directed repair process such that the heterologous sequence in the donor polynucleotide is integrated in-frame with the coding sequence of the targeted chromosomal sequence, thereby linking the heterologous coding sequence to an endogenous regulatory system. Components of the method are detailed below.

### (a) targeting endonuclease

The method comprises, in part, introducing into a cell at least one targeting endonuclease or nucleic acid encoding a targeting endonuclease. The targeting endonuclease may be a naturally-occurring protein or an engineered protein. In some embodiments, the targeting endonuclease may be a meganuclease or a homing endonuclease. In other embodiments, the targeting endonuclease may be a transcription activator-like effector (TALE)-nuclease. In preferred embodiments, the targeting endonuclease may be a zinc finger nuclease. Typically, a zinc finger nuclease comprises a DNA binding domain (i.e., zinc finger) and a cleavage domain (i.e., nuclease), which are described below.

### (i) zinc finger binding domain

Zinc finger binding domains may be engineered to recognize and bind to any nucleic acid sequence of choice. See, for example, Beerli et al. (2002) Nat. Biotechnol. 20:135-141; Pabo et al. (2001) Ann. Rev. Biochem. 70:313-340; Isalan et al. (2001) Nat. Biotechnol. 19:656-660; Segal et al. (2001) Curr. Opin. Biotechnol. 12:632-637; Choo et al. (2000) Curr. Opin. Struct. Biol. 10:411-416; Zhang et al. (2000) J. Biol. Chem. 275(43):33850-33860; Doyon et al. (2008) Nat. Biotechnol. 26:702-708; and Santiago et al. (2008) Proc. Natl. Acad. Sci. USA 105:5809-5814. An engineered zinc finger binding domain may have a novel binding specificity compared to a naturally-occurring zinc finger protein. Engineering methods include, but are not limited to, rational design and various types of selection. Rational design includes, for example, using databases comprising doublet, triplet, and/or quadruplet nucleotide sequences and individual zinc finger amino acid sequences, in which each doublet, triplet or quadruplet nucleotide sequence is associated with one or more amino acid sequences of zinc fingers which bind the particular triplet or quadruplet sequence. See, for example, U.S. Pat. Nos. 6,453,242 and 6,534,261, the disclosures of which are incorporated by reference herein in their entireties. As an example, the algorithm of described in US patent 6,453,242 may be used to design a zinc finger binding domain to target a preselected sequence. Alternative methods, such as rational design using a nondegenerate recognition code table may also be used to design a zinc finger binding domain to target a specific sequence (Sera et al. (2002) Biochemistry 41:7074-7081). Publically available web-based tools for identifying potential target sites in DNA sequences and designing zinc finger binding domains may be found at http://www.zincfingertools.org and http://bindr.gdcb.iastate.edu/ZiFiT/, respectively (Mandell et al. (2006) Nuc. Acid Res. 34:W516-W523; Sander et al. (2007) Nuc. Acid Res. 35:W599-W605).

A zinc finger binding domain may be designed to recognize and bind a DNA sequence ranging from about 3 nucleotides to about 21 nucleotides in length, or from about 8 to about 19 nucleotides in length. In general, the zinc finger binding domains of the zinc finger nucleases disclosed herein comprise at least three zinc finger recognition regions (i.e., zinc fingers). In one embodiment, the zinc finger binding domain may comprise four zinc finger recognition regions. In another embodiment, the zinc finger binding domain may comprise five zinc finger recognition regions. In still another embodiment, the zinc finger binding domain may comprise six zinc finger recognition regions. A zinc finger binding domain may be designed to bind to any suitable target DNA sequence. See for example, U.S. Pat. Nos. 6,607,882; 6,534,261 and 6,453,242, the disclosures of which are incorporated by reference herein in their entireties.

Exemplary methods of selecting a zinc finger recognition region may include phage display and two-hybrid systems, and are disclosed in U.S. Pat. Nos. 5,789,538; 5,925,523; 6,007,988; 6,013,453; 6,410,248; 6,140,466; 6,200,759; and 6,242,568; as well as WO 98/37186; WO 98/53057; WO 00/27878; WO 01/88197 and GB 2,338,237, each of which is incorporated by reference herein in its entirety. In addition, enhancement of binding specificity for zinc finger binding domains has been described, for example, in WO 02/077227.

Zinc finger binding domains and methods for design and construction of fusion proteins (and polynucleotides encoding same) are known to those of skill in the art and are described in detail in U.S. Patent Application Publication Nos. 20050064474 and 20060188987, each incorporated by reference herein in its entirety. Zinc finger recognition regions and/or multi-fingered zinc finger proteins may be linked together using suitable linker sequences, including for example, linkers of five or more amino acids in length. See, U.S. Pat. Nos. 6,479,626; 6,903,185; and 7,153,949, the disclosures of which are incorporated by reference herein in their entireties, for non-limiting examples of linker sequences of six or more amino acids in length. The zinc finger binding domain described herein may include a combination of suitable linkers between the individual zinc fingers of the protein.

In some embodiments, the zinc finger nuclease may further comprise a nuclear localization signal or sequence (NLS). A NLS is an amino acid sequence which facilitates targeting the zinc finger nuclease protein into the nucleus to introduce a double stranded break at the target sequence in the chromosome. Nuclear localization signals are known in the art. See, for example, Makkerh et al. (1996) Current Biology 6:1025-1027.

An exemplary zinc finger DNA binding domain recognizes and binds a sequence having at least about 80% sequence identity to a sequence chosen from SEQ ID NO:1, 2, 3, 4, 5, 6, 8, and 9. In other embodiments, the sequence identity may be about 81%, 82%, 83%, 84%, 85%, 86%, 87%, 88%, 89%, 90%, 91%, 92%, 93%, 94%, 95%, 96%, 97%, 98%, 99%, or 100%.

### (iii) cleavage domain

A zinc finger nuclease also includes a cleavage domain. The cleavage domain portion of the zinc finger nucleases disclosed herein may be obtained from any endonuclease or exonuclease. Non-limiting examples of endonucleases from which a cleavage domain may be derived include, but are not limited to, restriction endonucleases and homing endonucleases. See, for example, 2002-2003 Catalog, New England Biolabs, Beverly, Mass.; and Belfort et al. (1997) Nucleic Acids Res. 25:3379-3388 or www.neb.com. Additional enzymes that cleave DNA are known (e.g., S1 Nuclease; mung bean nuclease; pancreatic DNase I; micrococcal nuclease; yeast HO endonuclease). See also Linn et al. (eds.) Nucleases, Cold Spring Harbor Laboratory Press, 1993. One or more of these enzymes (or functional fragments thereof) may be used as a source of cleavage domains.

A cleavage domain also may be derived from an enzyme or portion thereof, as described above, that requires dimerization for cleavage activity. Two zinc finger nucleases may be required for cleavage, as each nuclease comprises a monomer of the active enzyme dimer. Alternatively, a single zinc finger nuclease may comprise both monomers to create an active enzyme dimer. As used herein, an "active enzyme dimer" is an enzyme dimer capable of cleaving a nucleic acid molecule. The two cleavage monomers may be derived from the same endonuclease (or functional fragments thereof), or each monomer may be derived from a different endonuclease (or functional fragments thereof).

When two cleavage monomers are used to form an active enzyme dimer, the recognition sites for the two zinc finger nucleases are preferably disposed such that binding of the two zinc finger nucleases to their respective recognition sites places the cleavage monomers in a spatial orientation to each other that allows the cleavage monomers to form an active enzyme dimer, e.g., by dimerizing. As a result, the near edges of the recognition sites may be separated by about 5 to about 18 nucleotides. For instance, the near edges may be separated by about 5, 6, 7, 8, 9, 10, 11, 12, 13, 14, 15, 16, 17 or 18 nucleotides. It will however be understood that any integral number of nucleotides or nucleotide pairs may intervene between two recognition sites (e.g., from about 2 to about 50 nucleotide pairs or more). The near edges of the recognition sites of the zinc finger nucleases, such as for example those described in detail herein, may be separated by 6 nucleotides. In general, the site of cleavage lies between the recognition sites.

Restriction endonucleases (restriction enzymes) are present in many species and are capable of sequence-specific binding to DNA (at a recognition site), and cleaving DNA at or near the site of binding. Certain restriction enzymes (e.g., Type IIS) cleave DNA at sites removed from the recognition site and have separable binding and cleavage domains. For example, the Type IIS enzyme Fokl catalyzes double-stranded cleavage of DNA, at 9 nucleotides from its recognition site on one strand and 13 nucleotides from its recognition site on the other. See, for example, U.S. Pat. Nos. 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Biol. Chem. 269:31, 978-31, 982. Thus, a zinc finger nuclease may comprise the cleavage domain from at least one Type IIS restriction enzyme and one or more zinc finger binding domains, which may or may not be engineered. Exemplary Type IIS restriction enzymes are described for example in International Publication WO 07/014,275, the disclosure of which is incorporated by reference herein in its entirety. Additional restriction enzymes also contain separable binding and cleavage domains, and these also are contemplated by the present disclosure. See, for example, Roberts et al. (2003) Nucleic Acids Res. 31:418-420.

An exemplary Type IIS restriction enzyme, whose cleavage domain is separable from the binding domain, is Fokl. This particular enzyme is active as a dimmer (Bitinaite et al. (1998) Proc. Natl. Acad. Sci. USA 95: 10, 570-10, 575). Accordingly, for the purposes of the present disclosure, the portion of the Fokl enzyme used in a zinc finger nuclease is considered a cleavage monomer. Thus, for targeted double-stranded cleavage using a Fokl cleavage domain, two zinc finger nucleases, each comprising a Fokl cleavage monomer, may be used to reconstitute an active enzyme dimer. Alternatively, a single polypeptide molecule containing a zinc finger binding domain and two Fokl cleavage monomers may also be used.

In certain embodiments, the cleavage domain may comprise one or more engineered cleavage monomers that minimize or prevent homodimerization, as described, for example, in U.S. Patent Publication Nos. 20050064474, 20060188987, and 20080131962, each of which is incorporated by reference herein in its entirety. By way of non-limiting example, amino acid residues at positions 446, 447, 479, 483, 484, 486, 487, 490, 491, 496, 498, 499, 500, 531, 534, 537, and 538 of Fokl are all targets for influencing dimerization of the Fokl cleavage half-domains. Exemplary engineered cleavage monomers of Fokl that form obligate heterodimers include a pair in which a first cleavage monomer includes mutations at amino acid residue positions 490 and 538 of Fokl and a second cleavage monomer that includes mutations at amino-acid residue positions 486 and 499.

Thus, in one embodiment, a mutation at amino acid position 490 replaces Glu (E) with Lys (K); a mutation at amino acid residue 538 replaces Iso (I) with Lys (K); a mutation at amino acid residue 486 replaces Gln (Q) with Glu (E); and a mutation at position 499 replaces Iso (I) with Lys (K). Specifically, the engineered cleavage monomers may be prepared by mutating positions 490 from E to K and 538 from I to K in one cleavage monomer to produce an engineered cleavage monomer designated "E490K:I538K" and by mutating positions 486 from Q to E and 499 from I to L in another cleavage monomer to produce an engineered cleavage monomer designated "Q486E:I499L." The above described engineered cleavage monomers are obligate heterodimer mutants in which aberrant cleavage is minimized or abolished. Engineered cleavage monomers may be prepared using a suitable method, for example, by site-directed mutagenesis of wild-type cleavage monomers (Fokl) as described in U.S. Patent Publication No. 20050064474 (see Example 5).

The zinc finger nuclease described above may be engineered to introduce a double stranded break at the targeted site of integration. The double stranded break may be at the targeted site of integration, or it may be up to 1, 2, 3, 4, 5, 10, 15, 20, 25, 30, 35, 40, 45, 50, 100, or 1000 nucleotides away from the site of integration. In some embodiments, the double stranded break may be up to 1, 2, 3, 4, 5, 10, 15, or 20 nucleotides away from the site of integration. In other embodiments, the double stranded break may be up to 10, 15, 20, 25, 30, 35, 40, 45, or 50 nucleotides away from the site of integration. In yet other embodiments, the double stranded break may be up to 50, 100, or 1000 nucleotides away from the site of integration.

### (iii) additional methods for targeted cleavage

Any nuclease having a target site in a chromosomal sequence may be used in the methods disclosed herein. For example, homing endonucleases and meganucleases have very long recognition sequences, some of which are likely to be present, on a statistical basis, once in a human-sized genome. Any such nuclease having a unique target site in a cellular genome may be used instead of, or in addition to, a zinc finger nuclease, for targeted cleavage of a cell chromosome.

Non-limiting examples of homing endonucleases include I-Scel, I-Ceul, PI-Pspl, PI-Sce, I-SceIV, I-CsmI, I-PanI, I-SceII, I-Ppol, I-SceIII, I-Crel, I-TevI, I-Tevll and I-TevIII. The recognition sequences of these enzymes are known in the art. See also U.S. Pat. No. 5,420,032; U.S. Pat. No. 6,833,252; Belfort et al. (1997) Nucleic Acids Res. 25:3379-3388; Dujon et al. (1989) Gene 82:115-118; Perler et al. (1994) Nucleic Acids Res. 22, 1125-1127; Jasin (1996) Trends Genet. 12:224-228; Gimble et al. (1996) J. Mol. Biol. 263:163-180; Argast et al. (1998) J. Mol. Biol. 280:345-353 and the New England Biolabs catalogue.

Although the cleavage specificity of most homing endonucleases is not absolute with respect to their recognition sites, the sites are of sufficient length that a single cleavage event per mammalian-sized genome may be obtained by expressing a homing endonuclease in a cell containing a single copy of its recognition site. It has also been reported that the specificity of homing endonucleases and meganucleases may be engineered to bind non-natural target sites. See, for example, Chevalier et al. (2002) Molec. Cell 10:895-905; Epinat et al. (2003) Nucleic Acids Res. 31:2952-2962; Ashworth et al. (2006) Nature 441:656-659; Paques et al. (2007) Current Gene Therapy 7:49-66.

### (iv) nucleic acid encoding a zinc finger nuclease

The zinc finger nuclease may be introduced into the cell as a nucleic acid that encodes the zinc finger nuclease. The nucleic acid encoding a zinc finger nuclease may be DNA or RNA. In one embodiment, the nucleic acid encoding a zinc finger nuclease may DNA. For example, plasmid DNA comprising a zinc finger nuclease coding sequence may be introduced into the cell. In another embodiment, the nucleic acid encoding a zinc finger nuclease may be RNA or mRNA. When the nucleic acid encoding a zinc finger nuclease is mRNA, the mRNA molecule may be 5' capped. Similarly, when the nucleic acid encoding a zinc finger nuclease is mRNA, the RNA molecule may be polyadenylated. Thus, a nucleic acid according to the method may be a capped and polyadenylated mRNA molecule encoding a zinc finger nuclease. Methods for capping and polyadenylating mRNA are known in the art.

### (b) donor polynucleotide

The method for integrating the heterologous coding sequence into a targeted chromosomal sequence further comprises introducing into the cell at least one donor polynucleotide comprising the heterologous coding sequence. A donor polynucleotide comprises not only the heterologous coding sequence, as detailed above in section (I)(a), but also comprises an upstream sequence and a downstream sequence. The upstream and downstream sequences flank the heterologous coding sequence in the donor polynucleotide. Furthermore, the upstream and downstream sequences share substantial sequence identity with either side of the site of integration in the chromosome.

The upstream and downstream sequences in the donor polynucleotide are selected to promote recombination between the targeted chromosomal sequence and the donor polynucleotide. The upstream sequence, as used herein, refers to a nucleic acid sequence that shares sequence similarity with the chromosomal sequence upstream of the targeted site of integration. Similarly, the downstream sequence refers to a nucleic acid sequence that shares sequence similarity with the chromosomal sequence downstream of the targeted site of integration. The upstream and downstream sequences in the donor polynucleotide may have about 75%, 80%, 85%, 90%, 95%, or 100% sequence identity with the targeted chromosomal sequence. In other embodiments, the upstream and downstream sequences in the donor polynucleotide may have about 95%, 96%, 97%, 98%, 99%, or 100% sequence identity with the targeted chromosomal sequence. In an exemplary embodiment, the upstream and downstream sequences in the donor polynucleotide may have about 99% or 100% sequence identity with the targeted chromosomal sequence.

An upstream or downstream sequence may comprise from about 20 bp to about 2500 bp. In one embodiment, an upstream or downstream sequence may comprise about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. An exemplary upstream or downstream sequence may comprise about 200 bp to about 2000 bp, about 600 bp to about 1000 bp, or more particularly about 700 bp to about 1000 bp.

Typically, the donor polynucleotide will be DNA. The donor polynucleotide may be a DNA plasmid, a bacterial artificial chromosome (BAC), a yeast artificial chromosome (YAC), a viral vector, a linear piece of DNA, a PCR fragment, a naked nucleic acid, or a nucleic acid complexed with a delivery vehicle such as a liposome or poloxamer. In one embodiment, the donor polynucleotide comprising the heterologous coding sequence may be a DNA plasmid. In another embodiment, the donor polynucleotide comprising the heterologous coding sequence may be a BAC.

One of skill in the art would be able to construct a donor polynucleotide as described herein using well-known standard recombinant techniques (see, for example, Sambrook et al., 2001 and Ausubel et al., 1996).

### (c) delivery to cell

The zinc finger nuclease or nucleic acid encoding the zinc finger nuclease and the donor polynucleotide detailed above in sections (II)(a) and (II)(b) are introduced into the cell. Suitable delivery methods include microinjection, electroporation, sonoporation, biolistics, calcium phosphate-mediated transfection, cationic transfection, liposome transfection, dendrimer transfection, heat shock transfection, nucleofection transfection, magnetofection, lipofection, impalefection, optical transfection, proprietary agent-enhanced uptake of nucleic acids, and delivery via liposomes, immunoliposomes, virosomes, or artificial virions. In one embodiment, the molecules may be introduced into a cell by nucleofection. In another embodiment the molecules may be introduced into the by microinjection. The molecules may be microinjected into the nucleus or the cytoplasm of the cell.

The ratio of the donor polynucleotide comprising the heterologous coding sequence to the zinc finger nuclease or nucleic acid encoding the zinc finger nuclease can and will vary. In general, the ratio of the donor polynucleotide to the zinc finger nuclease molecule may range from about 1:10 to about 10:1. In various embodiments, the ratio of donor polynucleotide to zinc finger nuclease molecules may be about 1:10, 1:9,1:8, 1:7, 1:6, 1:5, 1:4, 1:3, 1:2, 1:1, 2:1, 3:1, 4:1, 5:1, 6:1, 7:1, 8:1, 9:1, or 10:1. In one embodiment, the ratio may be about 1:1.

In embodiments in which more than one zinc finger nuclease molecule and more than one donor polynucleotide are introduced into a cell, the molecules may be introduced simultaneously or sequentially. For example, zinc finger nuclease molecules, each specific for a distinct recognition sequence, as well as the corresponding donor polynucleotides, may be introduced at the same time. Alternatively, each zinc finger molecule, as well as the corresponding donor polynucleotide, may be introduced sequentially.

### (d) culturing the cell

The method further comprises maintaining the cell under appropriate conditions such that the zinc finger nuclease-mediated integration may occur. The cell may be cultured using standard procedures to allow expression of the zinc finger nuclease. Standard cell culture techniques are described, for example, in Santiago et al. (2008) PNAS 105:5809-5814; Moehle et al. (2007) PNAS 104:3055-3060; Urnov et al. (2005) Nature 435:646-651; and Lombardo et al (2007) Nat. Biotechnology 25:1298-1306. Those of skill in the art appreciate that methods for culturing cells are known in the art and can and will vary depending on the cell type. Routine optimization may be used, in all cases, to determine the best techniques for a particular cell type.

In embodiments in which the cell is a one-cell embryo, the embryo may be cultured *in vitro* (e.g., in cell culture). Typically, the embryo is cultured at an appropriate temperature and in appropriate media with the necessary O₂/CO₂ ratio to allow the expression of the zinc finger nuclease. Suitable non-limiting examples of media include M2, M16, KSOM, BMOC, and HTF media. A skilled artisan will appreciate that culture conditions can and will vary depending on the species of embryo. Routine optimization may be used, in all cases, to determine the best culture conditions for a particular species of embryo. In some instances, the embryo also may be cultured *in vivo* by transferring the embryo into the uterus of a female host. Generally speaking the female host is from the same or similar species as the embryo. Preferably, the female host is pseudo-pregnant. Methods of preparing pseudo-pregnant female hosts are known in the art. Additionally, methods of transferring an embryo into a female host are known. Culturing an embryo *in vivo* permits the embryo to develop and may result in a live birth of an animal derived from the embryo.

During this step of the process, the zinc finger nuclease (which in some case is expressed from the introduced nucleic acid) recognizes, binds, and cleaves the target sequence in the chromosome. The double-stranded break introduced by the zinc finger nuclease is repaired, via homologous recombination with the donor polynucleotide, such that the heterologous coding sequence of the donor polynucleotide is integrated into the chromosomal location. The donor polynucleotide may be physically integrated or, alternatively, the donor polynucleotide may be used as a template for repair of the break, resulting in the integration of the heterologous coding sequence as well as all or part of the upstream and downstream sequences of the donor polynucleotide into the chromosome.

### (III) Method for Using an Endogenous Regulator System to Regulate Expression of Heterologous Protein(s)

Yet another aspect provides a method for using an endogenous regulatory system to regulate the expression of heterologous protein(s). The method comprises utilizing a cell comprising a chromosomally integrated sequence encoding at least one heterologous protein, which is detailed above in section (I), or integrating a sequence encoding at least one heterologous protein into a targeted chromosomal location, as detailed above in section (II). The method further comprises maintaining the cell under conditions such that the endogenous regulatory system is activated, and the endogenous and heterologous coding sequences are transcribed into a single transcript. Separate endogenous and heterologous protein(s) are produced during translation because of the presence of the 2A peptide(s). Thus, the expression of heterologous protein(s) is controlled by endogenous transcriptional regulatory mechanisms.

### (IV) Applications

The methods disclosed herein may be used for a variety of commercial, research, and clinical uses. Because the endogenous and heterologous sequences are transcribed into a transcript with one open reading frame, the amount of each protein produced is substantially similar. Thus, the level of heterologous protein(s) produced in the cell may be controlled by choosing the appropriate endogenous regulatory system. Furthermore, because endogenous regulatory systems are used to regulate their expression, the heterologous sequences typically are not subject to silencing effects.

The methods and cells provided herein may be used to produce large quantities of recombinant proteins that have a variety of commercial applications. The recombinant protein may be an antibody, a fragment of an antibody, a monoclonal antibody, an antibody heavy chain, an antibody light chain, a humanized antibody, a humanized monoclonal antibody, a chimeric antibody, a glycoprotein, an enzyme, a therapeutic protein, a nutraceutical protein, a vaccine, and a protein functioning as a blood factor, a thrombolytic agent, an anticoagulant, a hormone, a growth factor, an interferon or an interleukin. Additionally, the method and cells disclosed herein also may be used to deliver therapeutic proteins to a cell, such that the cell continually produces the therapeutic protein at the appropriate levels.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

When introducing elements of the present disclosure or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

A "gene," as used herein, refers to a DNA region (including exons and introns) encoding a gene product, as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites, and locus control regions.

A "heterologous protein" is a protein that is not native (i.e., foreign) to the cell or organism of interest.

The terms "nucleic acid" and "polynucleotide" refer to a deoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogs of natural nucleotides, as well as nucleotides that are modified in the base, sugar and/or phosphate moieties (e.g., phosphorothioate backbones). In general, an analog of a particular nucleotide has the same base-pairing specificity; i.e., an analog of A will base-pair with T.

The terms "polypeptide" and "protein" are used interchangeably to refer to a polymer of amino acid residues.

The term "recombination" refers to a process of exchange of genetic information between two polynucleotides. For the purposes of this disclosure, "homologous recombination" refers to the specialized form of such exchange that takes place, for example, during repair of double-strand breaks in cells. This process requires sequence similarity between the two polynucleotides, uses a "donor" or "exchange" molecule to template repair of a "target" molecule (i.e., the one that experienced the double-strand break), and is variously known as "non-crossover gene conversion" or "short tract gene conversion," because it leads to the transfer of genetic information from the donor to the target. Without being bound by any particular theory, such transfer can involve mismatch correction of heteroduplex DNA that forms between the broken target and the donor, and/or "synthesis-dependent strand annealing," in which the donor is used to resynthesize genetic information that will become part of the target, and/or related processes. Such specialized homologous recombination often results in an alteration of the sequence of the target molecule such that part or all of the sequence of the donor polynucleotide is incorporated into the target polynucleotide.

As used herein, the terms "target site" or "target sequence" refer to a nucleic acid sequence that defines a portion of a chromosomal sequence to be edited and to which a zinc finger nuclease is engineered to recognize and bind, provided sufficient conditions for binding exist.

Techniques for determining nucleic acid and amino acid sequence identity are known in the art. Typically, such techniques include determining the nucleotide sequence of the mRNA for a gene and/or determining the amino acid sequence encoded thereby, and comparing these sequences to a second nucleotide or amino acid sequence. Genomic sequences can also be determined and compared in this fashion. In general, identity refers to an exact nucleotide-to-nucleotide or amino acid-to-amino acid correspondence of two polynucleotides or polypeptide sequences, respectively. Two or more sequences (polynucleotide or amino acid) can be compared by determining their percent identity. The percent identity of two sequences, whether nucleic acid or amino acid sequences, is the number of exact matches between two aligned sequences divided by the length of the shorter sequences and multiplied by 100. An approximate alignment for nucleic acid sequences is provided by the local homology algorithm of Smith and Waterman, Advances in Applied Mathematics 2:482-489 (1981). This algorithm can be applied to amino acid sequences by using the scoring matrix developed by Dayhoff, Atlas of Protein Sequences and Structure, M. O. Dayhoff ed., 5 suppl. 3:353-358, National Biomedical Research Foundation, Washington, D.C., USA, and normalized by Gribskov, Nucl. Acids Res. 14(6):6745-6763 (1986). An exemplary implementation of this algorithm to determine percent identity of a sequence is provided by the Genetics Computer Group (Madison, Wis.) in the "BestFit" utility application. Other suitable programs for calculating the percent identity or similarity between sequences are generally known in the art, for example, another alignment program is BLAST, used with default parameters. For example, BLASTN and BLASTP can be used using the following default parameters: genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+GenBank CDS translations+Swiss protein+Spupdate+PIR. Details of these programs can be found on the GenBank website. With respect to sequences described herein, the range of desired degrees of sequence identity is approximately 80% to 100% and any integer value therebetween. Typically the percent identities between sequences are at least 70-75%, preferably 80-82%, more preferably 85-90%, even more preferably 92%, still more preferably 95%, and most preferably 98% sequence identity.

Alternatively, the degree of sequence similarity between polynucleotides can be determined by hybridization of polynucleotides under conditions that allow formation of stable duplexes between regions that share a degree of sequence identity, followed by digestion with single-stranded-specific nuclease(s), and size determination of the digested fragments. Two nucleic acid, or two polypeptide sequences are substantially similar to each other when the sequences exhibit at least about 70%-75%, preferably 80%-82%, more-preferably 85%-90%, even more preferably 92%, still more preferably 95%, and most preferably 98% sequence identity over a defined length of the molecules, as determined using the methods above. As used herein, substantially similar also refers to sequences showing complete identity to a specified DNA or polypeptide sequence. DNA sequences that are substantially similar can be identified in a Southern hybridization experiment under, for example, stringent conditions, as defined for that particular system. Defining appropriate hybridization conditions is within the skill of the art. See, e.g., Sambrook et al., supra; Nucleic Acid Hybridization: A Practical Approach, editors B. D. Hames and S. J. Higgins, (1985) Oxford; Washington, D.C.; IRL Press).

Selective hybridization of two nucleic acid fragments can be determined as follows. The degree of sequence identity between two nucleic acid molecules affects the efficiency and strength of hybridization events between such molecules. A partially identical nucleic acid sequence will at least partially inhibit the hybridization of a completely identical sequence to a target molecule. Inhibition of hybridization of the completely identical sequence can be assessed using hybridization assays that are well known in the art (e.g., Southern (DNA) blot, Northern (RNA) blot, solution hybridization, or the like, see Sambrook, et al., Molecular Cloning: A Laboratory Manual, Second Edition, (1989) Cold Spring Harbor, N.Y.). Such assays can be conducted using varying degrees of selectivity, for example, using conditions varying from low to high stringency. If conditions of low stringency are employed, the absence of non-specific binding can be assessed using a secondary probe that lacks even a partial degree of sequence identity (for example, a probe having less than about 30% sequence identity with the target molecule), such that, in the absence of non-specific binding events, the secondary probe will not hybridize to the target.

When utilizing a hybridization-based detection system, a nucleic acid probe is chosen that is complementary to a reference nucleic acid sequence, and then by selection of appropriate conditions the probe and the reference sequence selectively hybridize, or bind, to each other to form a duplex molecule. A nucleic acid molecule that is capable of hybridizing selectively to a reference sequence under moderately stringent hybridization conditions typically hybridizes under conditions that allow detection of a target nucleic acid sequence of at least about 10-14 nucleotides in length having at least approximately 70% sequence identity with the sequence of the selected nucleic acid probe. Stringent hybridization conditions typically allow detection of target nucleic acid sequences of at least about 10-14 nucleotides in length having a sequence identity of greater than about 90-95% with the sequence of the selected nucleic acid probe. Hybridization conditions useful for probe/reference sequence hybridization, where the probe and reference sequence have a specific degree of sequence identity, can be determined as is known in the art (see, for example, Nucleic Acid Hybridization: A Practical Approach, editors B. D. Hames and S. J. Higgins, (1985) Oxford; Washington, D.C.; IRL Press). Conditions for hybridization are well-known to those of skill in the art.

Hybridization stringency refers to the degree to which hybridization conditions disfavor the formation of hybrids containing mismatched nucleotides, with higher stringency correlated with a lower tolerance for mismatched hybrids. Factors that affect the stringency of hybridization are well-known to those of skill in the art and include, but are not limited to, temperature, pH, ionic strength, and concentration of organic solvents such as, for example, formamide and dimethylsulfoxide. As is known to those of skill in the art, hybridization stringency is increased by higher temperatures, lower ionic strength and lower solvent concentrations. With respect to stringency conditions for hybridization, it is well known in the art that numerous equivalent conditions can be employed to establish a particular stringency by varying, for example, the following factors: the length and nature of the sequences, base composition of the various sequences, concentrations of salts and other hybridization solution components, the presence or absence of blocking agents in the hybridization solutions (e.g., dextran sulfate, and polyethylene glycol), hybridization reaction temperature and time parameters, as well as, varying wash conditions. A particular set of hybridization conditions may be selected following standard methods in the art (see, for example, Sambrook, et al., Molecular Cloning: A Laboratory Manual, Second Edition, (1989) Cold Spring Harbor, N.Y.).

### EXAMPLES

The following examples are included to illustrate the invention.

### Example 1: Using the TUBA1 B Promoter to Express a Heterologous Protein

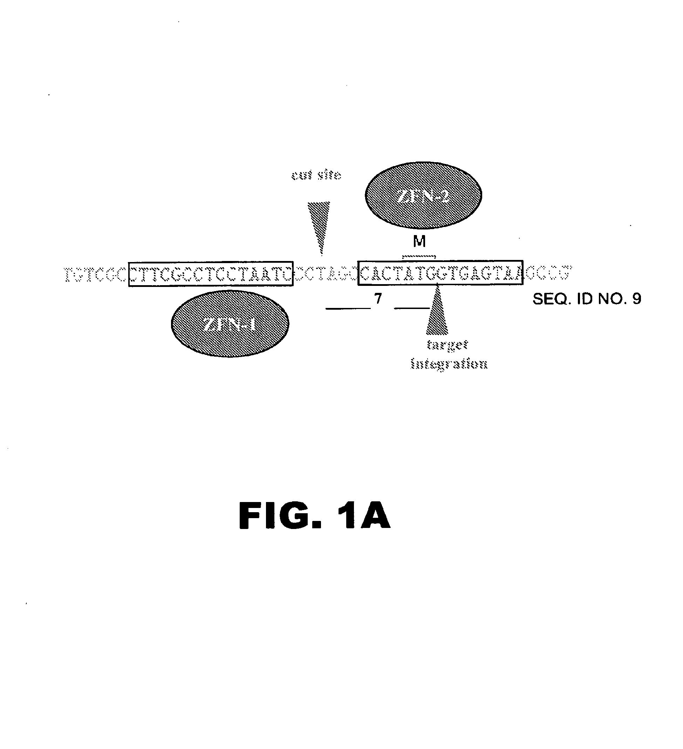

The following example details use of a tubulin promoter to regulate the expression of heterologous proteins. *TUBA1B,* which codes for tubulin alpha-1B, was chosen as the target chromosomal sequence. A pair of zinc finger nucleases (ZFNs) was designed to target a location in the human *TUBA1B* locus. For more details regarding ZFNs and methods of using to edit chromosomal regions see PCT/US2010/43167, the disclosure of which is incorporated by reference herein in its entirely. One ZFN was designed to bind the sequence 5' CTTCGCCTCCTAATC 3' (SEQ ID NO:1), and the other ZFN was designed to bind the sequence 5' CACTATGGTGAGTAA 3' (SEQ ID NO:2) (**FIG. 1A**). Upon binding, the ZFN pair introduces a double-stranded break in the sequence 5' CCTAGC 3' that lies between the two ZFN recognition sequences. Capped, polyadenylated mRNAs encoding the ZFN pair were produced using known molecular biology techniques.

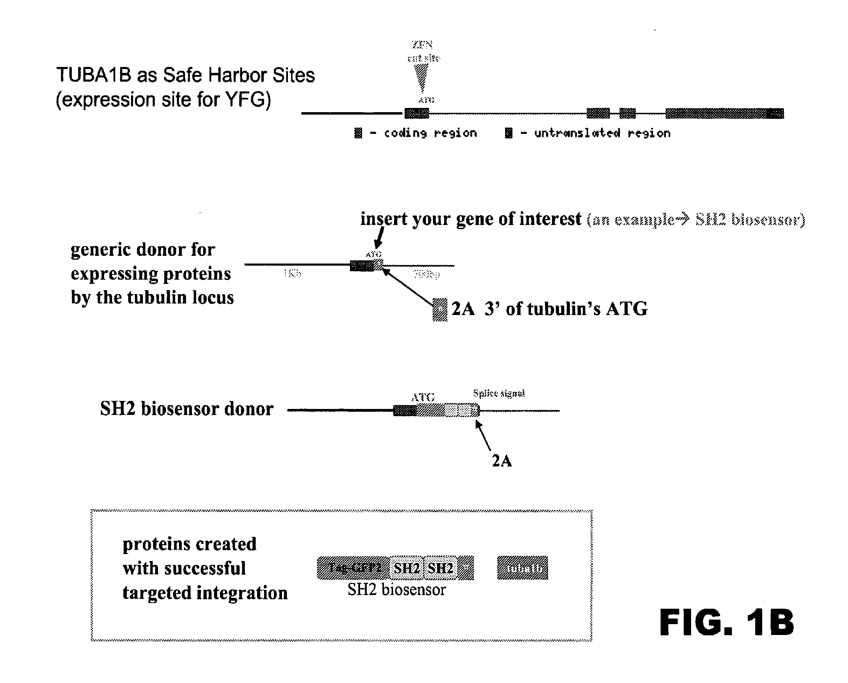

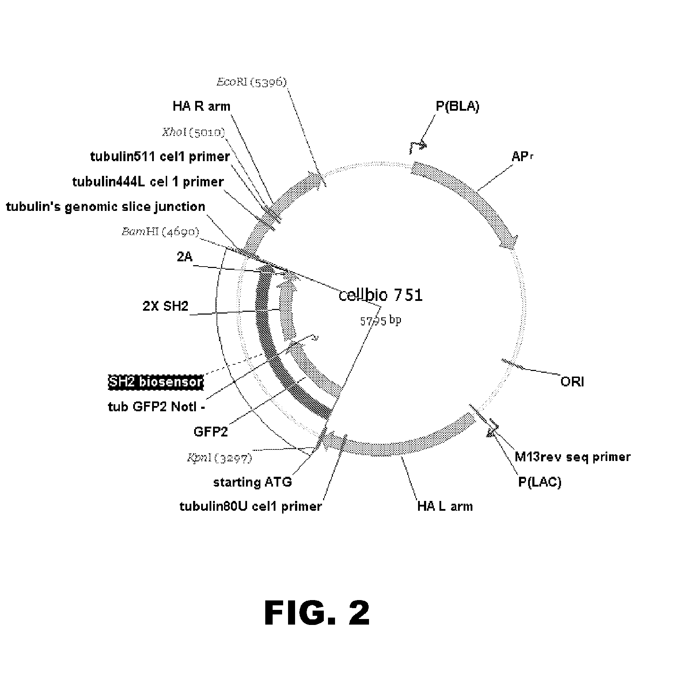

The gene of interest (i.e., SH2 biosensor) comprised a sequence encoding GFP linked to two SH2 domains (from Grb2 adaptor protein) and a 2A peptide domain (see **FIG. 1B**). A plasmid (**FIG. 2**) was constructed to serve as donor polynucleotide for the targeted integration of the SH2 biosensor sequence into the *TUBA1B* locus of human cell lines. The plasmid comprised the SH2 biosensor coding sequence flanked by 1 Kb and 700 bp of *TUBA1B* locus sequence upstream and downstream of the cut site introduced by the ZFN pair. The plasmid was designed such that the SH2 biosensor coding sequence would be integrated in-frame with the endogenous sequence just downstream of the tubulin start codon. Upon activation of the *TUBA1B* locus, two separate proteins are made, as depicted in **FIG. 1B****.**

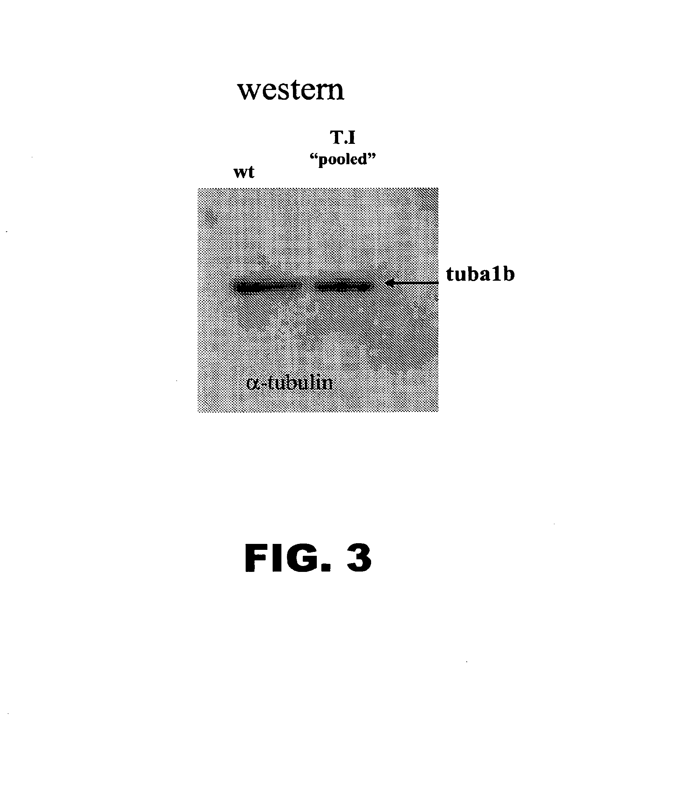

The donor plasmid and the pair of RNAs encoding ZFNs were transfected into U2OS, A549, K562, HEK293, or HEK293T cells. The nucleic acid mixture comprised one part donor DNA to one part ZFN RNAs. The transfected cells were then cultured under standard conditions. Analysis of individual cell clones revealed GFP fluorescence, indicating the expression of the heterologous biosensor. Western analysis confirmed that expression of α-tubulin was not affected by the targeted integration (**FIG. 3**).

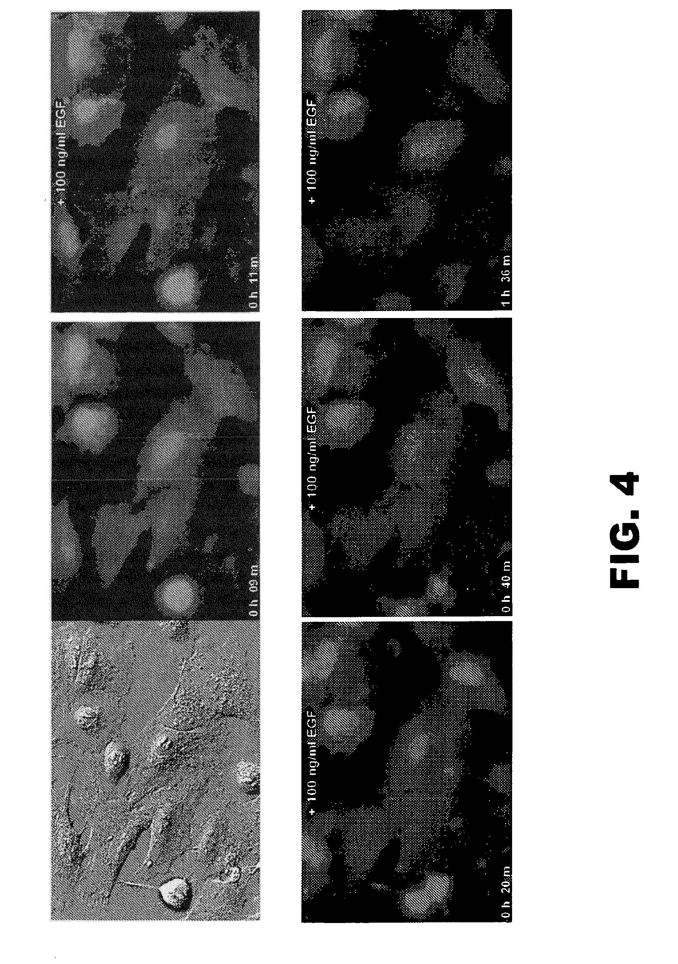

The SH2(Grb2)-containing biosensor is activated by EGF and undergoes nuclear translocation. A549 cells were transfected with the nucleic acids and cultured to allow integration and expression of the *TUBA1B* locus. Cells were exposed to 100 ng/ml of EGF and imaged. **FIG. 4** presents a time course of the nuclear translocation of the SH2 biosensor.

### Example 2: Using the ACTB Promoter to Express a Heterologous Protein

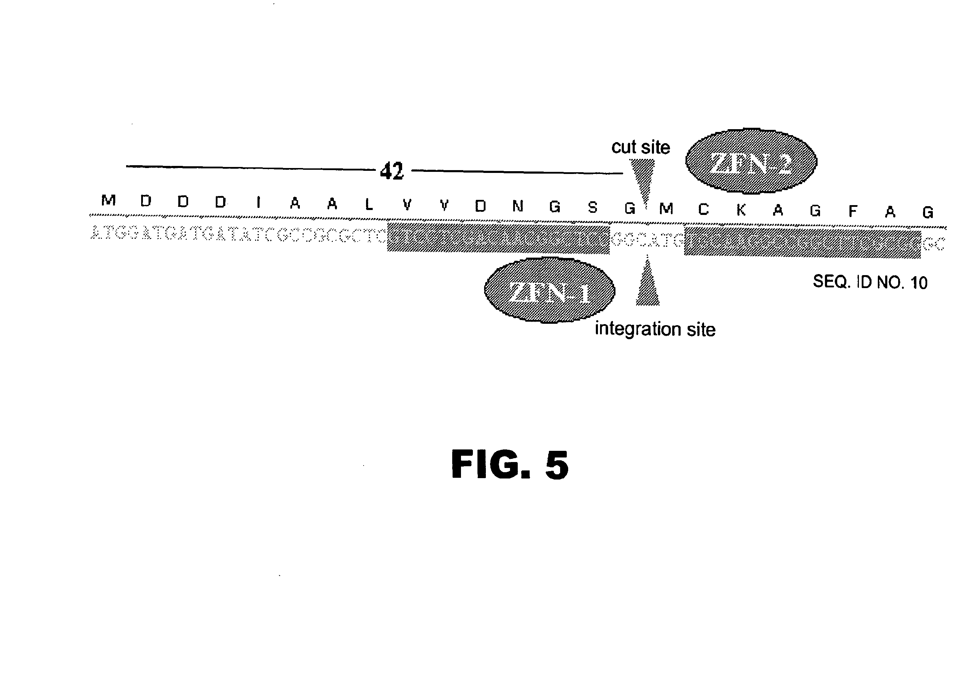

The following example was designed to test the use of a stronger promoter. A well known strong promoter is within the *ACTB* locus, which encodes β-actin. A pair of ZFNs was designed to target the human *ACTB* locus (**FIG. 5**). One ZFN was designed to bind the sequence 5' GTCGTCGACAACGGCTCC 3' (SEQ ID NO:3), and the other ZFN was designed to bind the sequence 5' TGCAAGGCCGGCTTCGCGG 3' (SEQ ID NO:4). Upon binding, the ZFN pair introduced a double-stranded break in the sequence 5' GGCATG 3' that lies between the two ZFN recognition sequences.

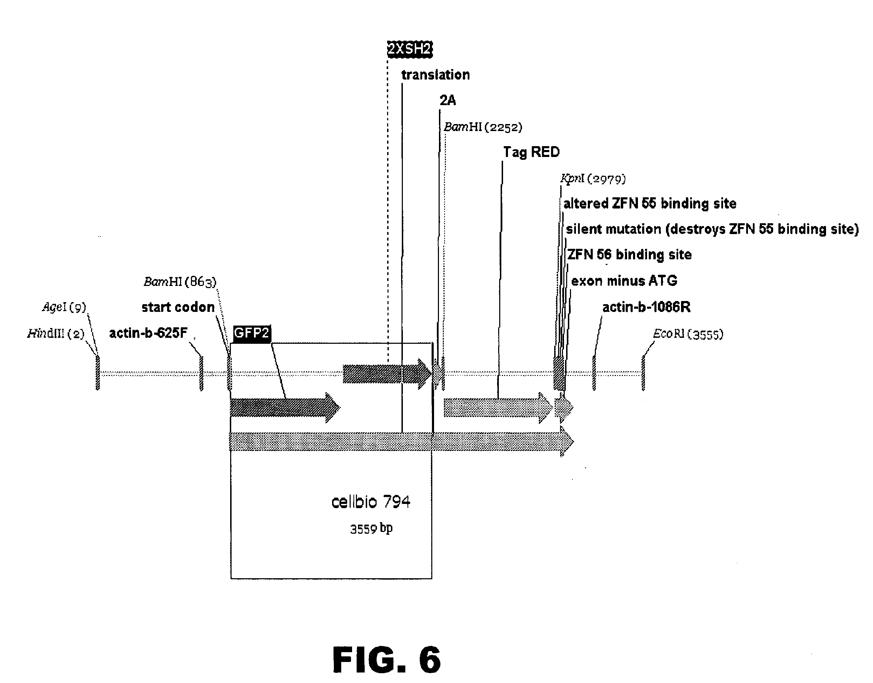

A donor plasmid was designed to provide the SH2 biosensor sequence, as well as tag the endogenously produced β-actin (i.e., GFP-2x-SH2(Grb2)-2A-RFP) (**FIG. 6**). The nucleic acids were introduced into cells, and two fluorescent proteins were made (i.e., GFP-SH2 biosensor and RFP-actin). The fluorescence of each protein was monitored using fluorescent microscopy.

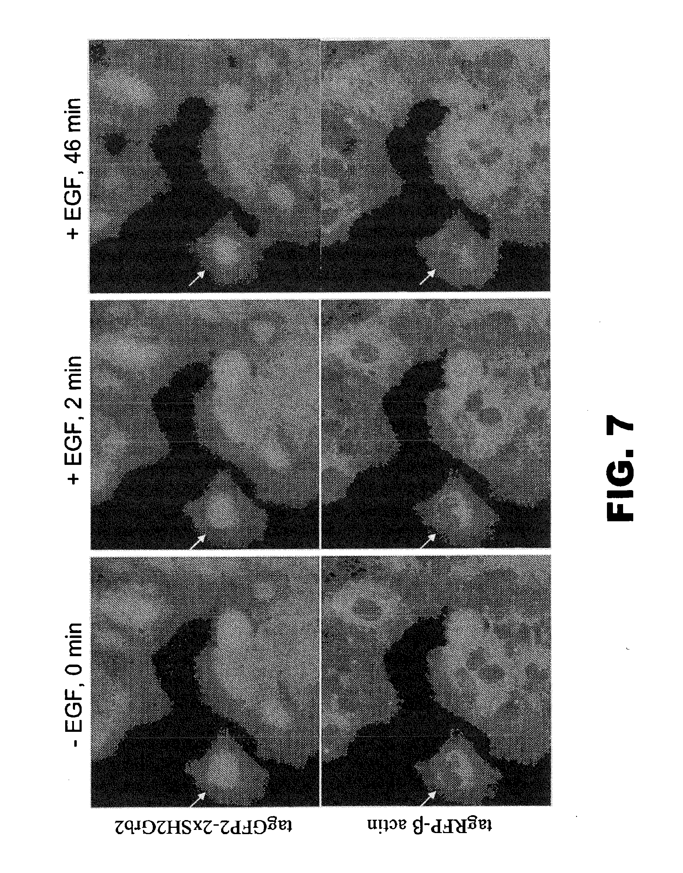

A549 cells were transfected with the nucleic acids and cultured to allow integration and expression of the *ACTB* locus. Cells were exposed to 100 ng/ml of EGF and imaged. **FIG. 7** presents a time course of the translocation of the GFP-Grb2 biosensor and the location of RFP-actin. The amount biosensor produced was so high that there were high levels of unbound or "free" biosensor, thereby drastically increasing the amount of background fluorescence.

### Example 3: Using the LMNB1 Promoter to Express a Heterologous Protein

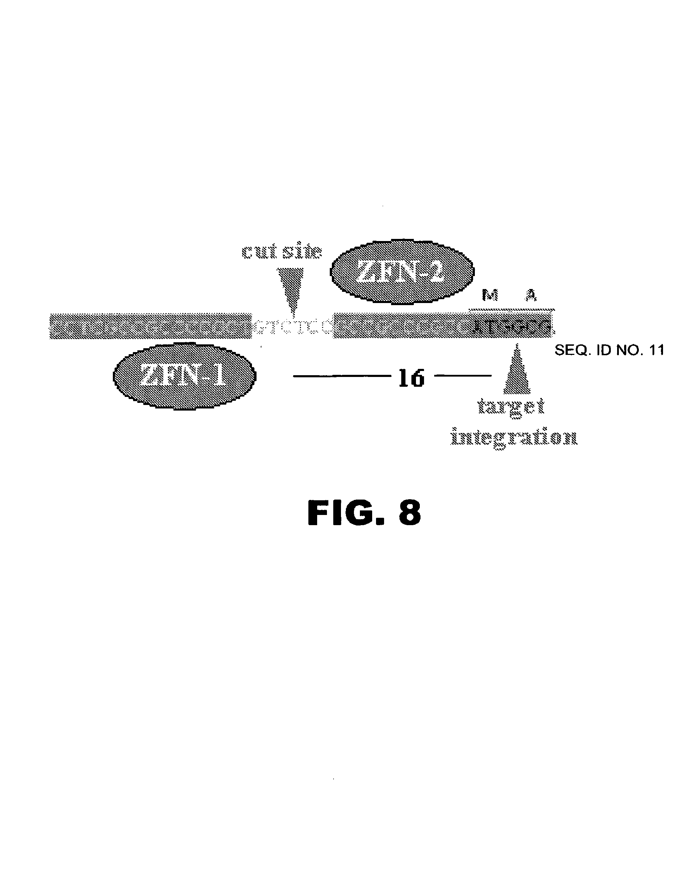

To target the *LMNB1* locus, which codes for lamin B1 protein, another pair of ZFNs was made (**FIG. 8**). One ZFN was designed to bind the sequence 5' CCTCGCCGCCCCGCT 3' (SEQ ID NO:5), and the other ZFN was designed to bind the sequence 5' GCCGCCCGCCATGGCG 3' (SEQ ID NO:6). Upon binding, the ZFN pair introduces a double-stranded break in the sequence 5' GTCTCC 3' that lies between the two recognition sequences.

A donor plasmid may be constructed to comprise a sequence encoding a heterologous protein that is flanked by *LMNB1* sequences upstream and downstream of the ZFN cleavage site. The nucleic acids encoding the ZFNs and the donor plasmid may be introduced into cells, and the cells may be monitored as detailed above.

### Example 4: Using the ACTB Promoter to Express Two Heterologous Proteins

This example was designed to determine whether two heterologous proteins could be expressed simultaneously from the same endogenous promoter. The *ACTB* locus was chosen for integration of sequences encoding secreted alkaline phosphatase (SEAP; ∼56 kD) and GFP (∼27 kD). These proteins were chosen because they are about the same size as the light and heavy chains of antibodies.

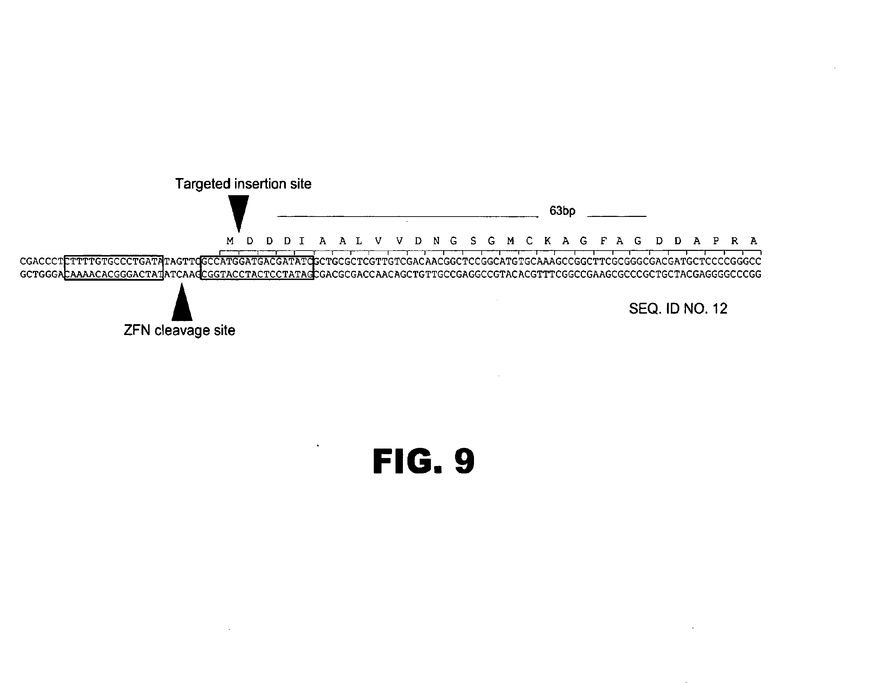

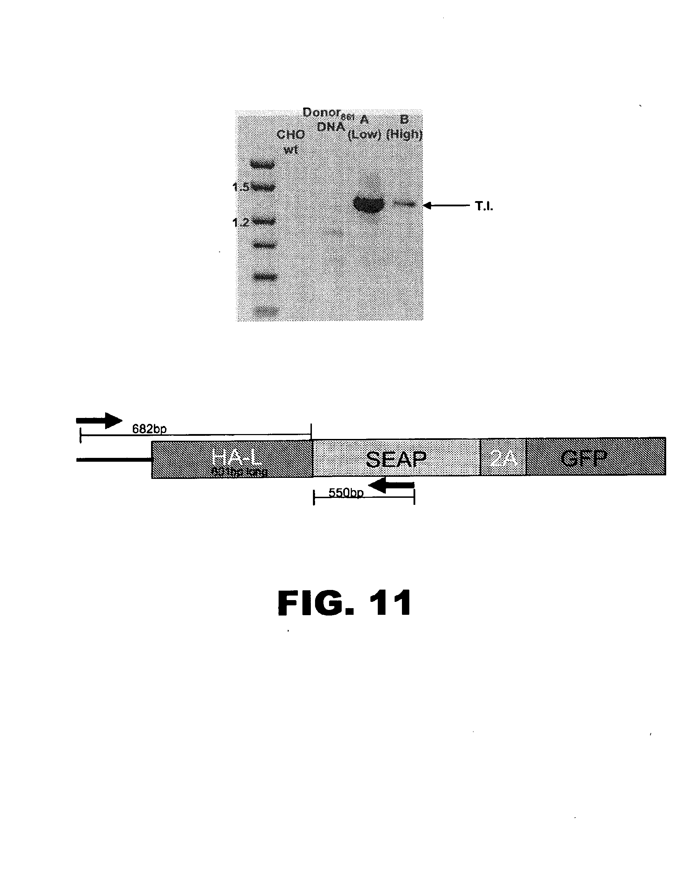

ZFNs were designed to target the *ACTB* locus of Chinese hamster ovary (CHO) cells (see **FIG. 9**) such that the heterologous sequence would be integrated just downstream of the start codon. One ZFN was designed to bind the sequence 5' CTTTTGTGCCCTGATA 3' (SEQ ID NO:7), and the other ZFN was designed to bind the sequence 5' GCCATGGATGACGATATC 3' (SEQ ID NO:8). Upon binding, the ZFN pair introduced a double-stranded break in the sequence 5' TAGTTC 3' that lies between the two recognition sequences. A donor plasmid was constructed that contained the sequence to be integrated (i.e., SEAP-2A-GFP), which was flanked by CHO *ACTB* sequences upstream and downstream of the ZFN cleavage site (**FIG**. **10****).** The nucleic acids encoding the ZFNs and the donor plasmid (at a low or high concentration) were nucleotransfected into CHO cells, and the cells were maintained as detailed above. The targeted integration was confirmed by junction PCR analysis using HAdet+2 and SEAP-500 primers which amplified a fragment of 1,232 base pairs as expected (**FIG. 11**).

A characteristic of CHO cells is that there is a high rate of random integration of donor DNA. To examine this, GFP was used to track targeted versus random insertions. Targeted integration at the *ACTB* locus yielded GFP-actin protein, which can be visualized in cells as green microfilaments. Random integrations gave rise to uniformly green cells with no localized GFP protein. Integration of sequence encoding SEAP and GFP into the *ACTB* locus U2OS cells (as detailed above in Example 2), however, resulted in a much higher ratio of targeted integrations versus random integrations.

It may be possible to eliminate random integrations in CHO cells by incorporating a suicide gene in the donor plasmid. Incorporation of the suicide gene will only occur by random integration. Due to the activity of the suicide gene, there will be no viable cell in case of random integration. Consequently, targeted integrant clones may be isolated.

### Example 5: Using the ACTB Promoter to Express Antibodies

CHO cells are frequently used for the production of therapeutic proteins such as antibodies. The SEAP coding sequence in the CHO donor plasmid detailed above may be exchanged for sequences encoding the light and heavy chains of an antibody. The sequence in the donor plasmid may be integrated into the *ACTB* locus of CHO cells as detailed above. The expressed antibody molecules may be purified from the CHO cells using standard procedures.

## Claims

1. A in vitro method for integrating a sequence encoding at least one heterologous protein into a chromosome of a cell such that expression of the at least one heterologous protein is regulated by an endogenous regulatory system, the method comprising:
a) introducing into the cell (i) at least one targeting endonuclease or nucleic acid encoding a targeting endonuclease, the targeting endonuclease being able to bind a target sequence and cut a cleavage site in a targeted chromosomal sequence that codes an endogenous protein chosen from the group consisting of an actin, a tubulin and a lamin; and (ii) at least one donor polynucleotide comprising the sequence encoding the at least one heterologous protein that is linked to a sequence encoding a 2A peptide to form a heterologous protein coding sequence, the heterologous protein coding sequence being flanked by an upstream sequence and a downstream sequence that have substantial sequence identity with either side of the cleavage site; and
b) maintaining the cell under conditions such that a double-stranded break introduced into the targeted chromosomal sequence by the targeting endonuclease is repaired by a homology-directed repair process such that the heterologous protein coding sequence in the donor polynucleotide is integrated in-frame into the targeted chromosomal sequence, whereby expression of the at least one heterologous protein is regulated by the endogenous regulatory system that regulates expression of the endogenous protein.

2. The method of claim 1, wherein the targeted endonuclease is a zinc finger nuclease.

3. The method of claim 1, wherein the targeting endonuclease is a zinc finger nuclease that binds a sequence having at least about 80% sequence identity to a sequence chosen from SEQ ID NO:7, 8, 3, 4, 1, 2, 5 and 6.

4. The method of claim 1, wherein the sequence encoding the 2A peptide is linked 5' or 3' to the sequence encoding the heterologous protein.

5. The method of claim 1, wherein the heterologous protein coding sequence is integrated near the beginning or near the end of the protein coding sequence of the targeted chromosomal sequence.

6. The method of claim 1, wherein the at least one heterologous protein is a heavy chain or a light chain of an antibody.

7. The method of claim 1, wherein the cell is a human cell or a mammalian cell.

8. The method of claim 3, wherein the sequence identity is about 85%, 90%, 95%, 99%, or 100%.

9. The method of claim 3, wherein the cell is a Chinese hamster ovary (CHO) cell, the targeted chromosomal sequence encodes an actin protein, and the zinc finger nuclease binds to a sequence chosen from SEQ ID NO: 7 and SEQ ID NO:8.

10. A cell comprising a chromosomally integrated sequence encoding at least one heterologous protein, the sequence encoding the at least one heterologous protein being integrated in-frame with a chromosomal sequence encoding an endogenous protein chosen from the group consisting of an actin, a tubulin and a lamin, such that expression of the at least one heterologous protein is coordinately controlled with expression of the endogenous protein.

11. The cell of claim 10, wherein each of the endogenous and heterologous proteins is produced as a discrete entity.

12. A in vitro method for using an endogenous regulatory system to regulate expression of at least one heterologous protein, the method comprising:
a) providing a cell comprising a chromosomally integrated sequence encoding at least one heterologous protein linked to a sequence encoding a 2A peptide, the sequences encoding the heterologous protein and the 2A peptide being integrated in-frame with a chromosomal sequence encoding an endogenous protein chosen from the group consisting of an actin, a tubulin and a lamin; and
b) maintaining the cell under conditions such that activation of the endogenous regulatory system produces one transcript encoding the heterologous protein, the 2A peptide, and the endogenous protein, wherein the 2A peptide disrupts translation such that each of the heterologous and endogenous proteins is produced as a discrete entity.

13. The method of claim 12, wherein the cell is a Chinese hamster ovary (CHO) cell, the chromosomal sequence encodes an actin protein, and the heterologous protein is a heavy chain or a light chain of an antibody.

## Patentansprüche

1. *In vitro*-Verfahren zur Integration einer wenigstens ein heterologes Protein codierenden Sequenz in ein Chromosom einer Zelle, so dass die Expression des wenigstens einen heterologen Proteins von einem endogenen Regulierungssystem geregelt wird, umfassend:
a) das Einführen in die Zelle von
(i) zumindest einer zielgerichteten Endonuklease oder einer eine zielgerichtete Endonuklease codierenden Nukleinsäure, wobei die zielgerichtete Endonuklease dazu fähig ist, an eine Zielsequenz zu binden und eine Spaltungsstelle in eine anvisierte chromosomale Sequenz zu schneiden, wobei die anvisierte chromosomale Sequenz ein endogenes Protein codiert, das aus der Gruppe, bestehend aus einem Actin, einem Tubulin und einem Lamin, ausgewählt wird; und
(ii) zumindest einem Donor-Polynukleotid, das die das wenigstens eine heterologe Protein codierende Sequenz umfasst, die mit einer ein 2A-Peptid codierenden Sequenz verbunden ist, um eine ein heterologes Protein codierende Sequenz zu bilden, wobei die das heterologe Protein codierende Sequenz von einer stromaufwärts liegenden Sequenz und einer stromabwärts liegenden Sequenz flankiert wird, die eine wesentliche Sequenzübereinstimmung mit beiden Seiten der Spaltungsstelle aufweisen; und
b) das Bewahren der Zelle unter derartigen Bedingungen, dass ein doppelsträngiger Bruch, der von der zielgerichteten Endonuklease in die anvisierte chromosomale Sequenz eingefügt wurde, derart von einem Homologie-gelenkten Reparaturprozess repariert wird, dass die das heterologe Protein codierende Sequenz im Donor-Polynukleotid in-frame in die anvisierte chromosomale Sequenz eingefügt wird, wobei die Expression des wenigstens einen heterologen Proteins von dem endogenen Regulierungssystem reguliert wird, das die Expression des endogenen Proteins reguliert.

2. Verfahren nach Anspruch 1, wobei die zielgerichtete Endonuklease eine Zinkfingernuklease ist.

3. Verfahren nach Anspruch 1 wobei die zielgerichtete Endonuklease eine Zinkfingernuklease ist, die an eine Sequenz bindet, die eine Sequenzübereinstimmung von mindestens 80 % mit einer der Sequenzen nach SEQ ID NO: 7, 8, 3, 4, 1, 2, 5 und 6 aufweist.

4. Verfahren nach Anspruch 1 wobei die das 2A-Peptid codierende Sequenz an die das heterologe Protein codierende Sequenz entweder 5'- oder 3'-gebunden ist.

5. Verfahren nach Anspruch 1, wobei die das heterologe Protein codierende Sequenz in der Nähe des Anfangs oder in der Nähe des Endes der Protein-codierenden Sequenz der anvisierten chromosomalen Sequenz eingefügt wird.

6. Verfahren nach Anspruch 1, wobei das wenigstens eine heterologe Protein eine schwere Kette oder eine leichte Kette eines Antikörpers ist.

7. Verfahren nach Anspruch 1, wobei die Zelle eine menschliche Zelle oder eine Säugetierzelle ist.

8. Verfahren nach Anspruch 3, wobei die Sequenzübereinstimmung ungefähr 85 %, 90 %, 95 %, 99 % oder 100 % beträgt.

9. Verfahren nach Anspruch 3, wobei die Zelle eine Chinese Hamster Ovary (CHO)-Zelle ist, die anvisierte chromosomale Sequenz ein Actin-Protein codiert und die Zinkfingernuklease an eine der Sequenzen nach SEQ ID NO: 7 und SEQ ID NO: 8 bindet.

10. Zelle, umfassend eine in das Chromosom eingefügte wenigstens ein heterologes Protein codierende Sequenz, wobei die wenigstens ein heterologes Protein codierende Sequenz derart in-frame mit einer chromosomalen Sequenz eingefügt ist, die ein endogenes Protein codiert, das aus der Gruppe, bestehend aus einem Actin, einem Tubulin und einem Lamin, ausgewählt ist, dass die Expression des wenigstens einen heterologen Proteins koordiniert mit der Expression des endogenen Proteins kontrolliert wird.

11. Zelle nach Anspruch 10, wobei jedes endogene und heterologe Protein als eine eigenständige Einheit produziert wird.

12. *In vitro*-Verfahren zur Nutzung eines endogenen Regulierungssystems zur Regulierung der Expression von wenigstens einem heterologen Protein, umfassend:
a) das Bereitstellen einer Zelle, wobei die Zelle eine in das Chromosom eingefügte Sequenz umfasst, die wenigstens ein heterologes Protein codiert und die mit einer ein 2A-Peptid codierenden Sequenz verbunden ist, wobei die das heterologe Protein und das 2A-Protein codierenden Sequenzen in-frame mit einer chromosomalen Sequenz eingefügt sind, die ein endogenes Protein codiert, das aus der Gruppe, bestehend aus einem Actin, einem Tubulin und einem Lamin, ausgewählt ist; und
b) das Bewahren der Zelle unter derartigen Bedingungen, dass die Aktivierung des endogenen Regulierungssystems die Produktion eines das heterologe Protein, das 2A-Peptid und das endogene Protein codierenden Transkripts herbeiführt, wobei das 2A-Peptid die Translation derart stört, dass jedes der heterologen und endogenen Proteine als eine eigenständige Einheit produziert wird.

13. Verfahren nach Anspruch 12, wobei die Zelle eine Chinese Hamster Ovary (CHO)-Zelle ist, die chromosomale Sequenz ein Actin-Protein codiert und das heterologe Protein eine schwere Kette oder eine leichte Kette eines Antikörpers ist.

## Revendications

1. Procédé *in vitro* permettant d'intégrer une séquence codant pour au moins une protéine hétérologue dans un chromosome d'une cellule de telle manière que l'expression de la au moins une protéine hétérologue est régulée par un système régulateur endogène, le procédé comprenant :
a) l'introduction dans la cellule (i) d'au moins une endonucléase de ciblage ou d'au moins un acide nucléique codant pour une endonucléase de ciblage, l'endonucléase de ciblage étant capable de lier une séquence cible et de couper un site de clivage dans une séquence chromosomique ciblée qui code pour une protéine endogène choisie dans le groupe constitué d'une actine, une tubuline et une lamine ; et (ii) d'au moins un polynucléotide donneur comprenant la séquence codant pour la au moins une protéine hétérologue qui est liée à une séquence codant pour un peptide 2A pour former une séquence codante de protéine hétérologue, la séquence codante de protéine hétérologue étant flanquée d'une séquence en amont et d'une séquence en aval qui présentent une identité de séquence substantielle avec l'un ou l'autre côté du site de clivage ; et
b) le maintien de la cellule dans des conditions telles qu'une rupture double brin introduite dans la séquence chromosomique ciblée par l'endonucléase de ciblage est réparée par un procédé de réparation dirigée par l'homologie de telle manière que la séquence codante de protéine hétérologue dans le polynucléotide donneur est intégrée dans le cadre dans la séquence chromosomique ciblée, moyennant quoi l'expression de la au moins une protéine hétérologue est régulée par le système régulateur endogène qui régule l'expression de la protéine endogène.

2. Procédé selon la revendication 1, dans lequel l'endonucléase ciblée est une nucléase à doigt de zinc.

3. Procédé selon la revendication 1, dans lequel l'endonucléase de ciblage est une nucléase à doigt de zinc qui lie une séquence présentant au moins environ 80 % d'identité de séquence avec une séquence choisie parmi SÉQ ID NO : 7, 8, 3, 4, 1, 2, 5 et 6.

4. Procédé selon la revendication 1, dans lequel la séquence codant pour le peptide 2A est liée en 5' ou en 3' par rapport à la séquence codant pour la protéine hétérologue.

5. Procédé selon la revendication 1, dans lequel la séquence codante de la protéine hétérologue est intégrée près du début ou près de la fin de la séquence codante de la protéine de la séquence chromosomique ciblée.

6. Procédé selon la revendication 1, dans lequel la au moins une protéine hétérologue est une chaîne lourde ou une chaîne légère d'un anticorps.

7. Procédé selon la revendication 1, dans lequel la cellule est une cellule humaine ou une cellule de mammifère.

8. Procédé selon la revendication 3, dans lequel l'identité de séquence est d'environ 85 %, 90 %, 95 %, 99 % ou 100 %.

9. Procédé selon la revendication 3, dans lequel la cellule est une cellule d'ovaire de hamster de Chine (CHO), la séquence chromosomique ciblée code pour une protéine actine, et la nucléase à doigt de zinc se lie à une séquence choisie parmi SÉQ ID NO : 7 et SÉQ ID NO : 8.

10. Cellule comprenant une séquence intégrée au niveau d'un chromosome codant pour au moins une protéine hétérologue, la séquence codant pour la au moins une protéine hétérologue étant intégrée dans le cadre avec une séquence chromosomique codant pour une protéine endogène choisie dans le groupe constitué d'une actine, une tubuline et une lamine, de telle manière que l'expression de la au moins une protéine hétérologue est contrôlée de façon coordonnée avec l'expression de la protéine endogène.

11. Cellule selon la revendication 10, dans laquelle chacune des protéines endogène et hétérologue est produite sous la forme d'une entité distincte.

12. Procédé *in vitro* permettant d'utiliser un système régulateur endogène pour réguler l'expression d'au moins une protéine hétérologue, le procédé comprenant :
a) la fourniture d'une cellule comprenant une séquence intégrée au niveau d'un chromosome codant pour au moins une protéine hétérologue liée à une séquence codant pour un peptide 2A, les séquences codant pour la protéine hétérologue et le peptide 2A étant intégrées dans le cadre avec une séquence chromosomique codant pour une protéine endogène choisie dans le groupe constitué d'une actine, une tubuline et une lamine ; et
b) le maintien de la cellule dans des conditions telles que l'activation du système régulateur endogène produit un transcrit codant pour la protéine hétérologue, le peptide 2A, et la protéine endogène, où le peptide 2A perturbe la traduction de telle manière que chacune des protéines hétérologue et endogène est produite sous la forme d'une entité distincte.

13. Procédé selon la revendication 12, dans lequel la cellule est une cellule d'ovaire de hamster de Chine (CHO), la séquence chromosomique code pour une protéine actine, et la protéine hétérologue est une chaîne lourde ou une chaîne légère d'un anticorps.
